**Europäisches Patentamt**

**European Patent Office**

**Office européen des brevets**

(19)

(11) Numéro de publication: **0 064 111**
**B1**

(12)

# FASCICULE DE BREVET EUROPEEN

(45) Date de publication du fascicule du brevet:
**05.02.86**

(51) Int. Cl.⁴: **A 61 B 3/10**

(21) Numéro de dépôt: **81400701.9**

(22) Date de dépôt: **05.05.81**

(54) **Dispositif pour observer et/ou photographier l'oeil d'un patient en vue de l'adaption d'une lentille de contact.**

(43) Date de publication de la demande:
**10.11.82 Bulletin 82/45**

(45) Mention de la délivrance du brevet:
**05.02.86 Bulletin 86/6**

(84) Etats contractants désignés:
**AT BE CH DE FR GB IT LI LU NL SE**

(56) Documents cités:
**DE - A - 2 410 021**
**FR - A - 2 213 046**
**GB - A - 2 018 413**
**US - A - 3 169 459**
**US - A - 3 842 253**

(73) Titulaire: **Duparchy, Jacques, 10 Rue Alquier Bouffard,
F-81100 Castres (FR)**

(72) Inventeur: **Duparchy, Jacques, 10 Rue Alquier Bouffard,
F-81100 Castres (FR)**

(74) Mandataire: **Ravina, Bernard, Cabinet Bernard
RAVINA 24, boulevard Riquet, F-31000 Toulouse (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit
être formée par écrit et motivée. Elle n'est réputée formée qu'après paiement de la taxe d'opposition (Art. 99(1) Convention sur le
brevet européen).

LIBER, STOCKHOLM 1986

## Description

L'invention concerne un dispositif pour observer l'oeil d'un patient en vue de l'adaptation d'une lentille de contact ou pour enregistrer par prise de vue une image de l'oeil.

Ce dispositif comporte une source d'éclairage en lumière blanche, pour éclairer l'oeil en lumière blanche, une source d'éclairage en lumière ultraviolette pour éclairer l'oeil en lumière ultraviolette lorsque dans celui-ci est instillé une substance fluorescente qui lorsqu'elle est excitée par la lumière ultraviolette émet des rayons lumineux visibles et un dispositif d'observation pour observer ou photographier l'oeil.

Par lentille de contact ou entend un élément en forme de cuvette qui est apposé de façon amovible sur l'oeil dans un but thérapentique, ou dans un but esthétique ou dans le but de corriger la vision du patient.

Par prise de vue an entend tous les procédés et moyens permettant l'enregistrement d'une ou plusieurs images et plus particulièrement les procédés et appareils photographiques.

Le port de lentilles de contact souples ou rigides sans nuisances pour le métabolisme cornéen ou conjonctival suppose résolus les problèmes d'intolérances.

Pour pallier à ces problèmes l'homme de l'art lors de la pose des prothèse procède à divers examens de tolérances, c'est ainsi qu'il teste la qualité et la quantité de larmes, contrôle le centrage et les déplacements de la prothèse sur l'oeil et observe le film lacrymal présent sous la prothèse.

Certains de ces examens sont effectués à l'aide d'une source d'éclairage émettant des rayons ultra-violets et visibles notamment dans les bleus qui excitent une substance fluorescente instillée dans l'oeil pour émettre un rayonnement visible.

De ce fait on visualise la circulation du film lacrymal sous lentille et les zones de faibles quantités lacrymales.

Malgré les résultats satisfaisants de ces examens, les lentilles de contact peuvent être à la longue mal tolérées par le sujet qui les porte.

En outre, l'homme de l'art ne peut jamais déterminer avec certitude la type de prothèse convenant le mieux à chaque sujet.

Il peut se présenter le cas ou des prothèses assez bien tolérées puissent être remplacées par d'autres d'un type différent conduisant à de meilleurs résultats de tolérance.

Pour pallier à ces différents inconvénients, il est apparu nécessaire de constituer un dossier de résultats des différents examens de tolérance avec des prothèses différentes par leur forme et leurs matériaux et ceci après leur port effectif.

Par analyse des différents résultats, l'homme de l'art sera en mesure de déterminer avec précision le type de lentilles qui sera le mieux toléré.

Les différents résultats de tolérance effectués pour chaque lentillede contact peuvent être mémorisés par prise de vue photographique.

Jusqu'à présent la prise de vue photographique et/ou l'observation de l'oeil équipé de sa lentille pose des problèmes majeurs.

Au moment de la prise de vue, l'homme de l'art doit utiliser une source d'éclairage constituée par une lampe et/ou un tube à éclat dont une partie des rayonnements se reflète sur la surface photographiée et/ou observée masquant ainsi certaines zones nécessaire à une interprétation correcte du résultat.

Les ophtalmomètres connus ne sont pas équipés en combinaison avec leur dispositif d'optique de moyens aptes à supprimer de l'image de l'oeil et sa prothèse oculaire les reflets dus aux sources d'éclairage et notamment ceux à la source d'éclaire en lumière ultraviolette.

On connaît un ophtalmomètre objet du brevet US 3.842.253 et Fr 2.213.046 qui présente l'avantage d'éclairer l'oeil et la leutille de contact soit en lumière ultraviolette, soit en lumière blanche.

Dans ce but cet appareil comporte une source d'éclairage qui est constituée par une lampe à incandescence et un filtre ultra-violet monté mobile de façon a être interposé sur le trajet des rayons lumineux, entre l'ampoule à incandescence et l'oeil du patient, ou à être dégagé de ce trajet selon que le patient désire éclairer l'oeil et la leutille de contact en lumière ultraviolette ou en lumière blanche.

La lumière ultraviolette excite une substance fluorescente précédemment instillée dans l'oeil.

Cette excitation se traduit par l'émission d'une lumière visible par exemple verte.

L'observateur qui observe l'oeil et la prothèse éclairés en lumière ultraviolette ou en lumière blanche est géné dans son observation en raison des reflets qui apparaissent, ces reflets étant dus à la source d'éclairage.

En raison de l'imperfection du filtre ultraviolet il apparait sur l'oeil, lorsque celui-ci est éclairé en lumière ultraviolette des reflets bleus.

Lorsque l'oeil est éclairé en lumière blanche ces reflets sont blancs. On conçoit aisément que les reflets qui apparaissent empêchent la vision ingégrale de l'oeil.

La présente invention a pour objet de pallier les inconvénients précédemment cités en mettant en oeuvre un dispositif équipé de moyens aptes à supprimer les reflets dus aux sources d'éclairage et notamment ceux dus à la source d'éclairage en lumière ultraviolette.

A cet effet, le dispositif selon l'invention pour observer et/ou photographier l'oeil d'un patient en vue de l'adaptation d'une lentille de contact du type de ceux comportant un dispositif d'observation pour observer et/ou photographier l'oeil, une source dè lumière blanche pour éclairer l'oeil en lumière blanche et une source de lumière ultraviolette pour éclairer l'oeil en lumière ultraviolette lorsque dans celui-ci est instillé une substance fluorescente qui lorsqu'elle est excitée par la lumière ultra violette émet des rayons lumineux dont la longueur d'onde est supérieure à une valeur $\lambda$ 1 égale à 500 nm, la dite source de lumière ultraviolette étant constituée

2

notamment par une lampe, par un tube à éclat et par un filtre ultraviolet qui ne laisse passer que la lumière ultraviolette, caractérisé en ce que le dispositif d'optique est équipé d'un filtre qui ne selectionne que les rayons issus de l'oeil dont la longueur d'onde est supérieure à λ 1 en sorte de supprimer les reflets dus à la source d'éclairage en lumière ultraviolette lorsque celle-ci éclaire l'oeil et la prothèse oculaire.

D'autres avantages et caractéristiques de l'invention apparaîtront à la lecture de la description d'une forme préférentielle de réalisation en se référant aux dessins annexés en lesquels :
- Les figures 1 et 2 sont des schémas illustrant le principe de fonctionnement du dispositif selon l'invention,
- la figure 3 est une vue schématique du dispositif selon l'invention,
- les figures 4 et 5 sont des vues de détail d'une autre forme de réalisation du dispositif selon l'invention,
- la figure 6 est une vue en plan d'une forme préférentielle de réalisation du dispositif,
- la figure 7 est une vue schématique d'une source d'éclairage en lumière blanche,
- la figure 8 est une vue en coupe selon la ligne BB de la figure 6.
- la figure 9 est une vue en perspective du dispositif selan l'invention.

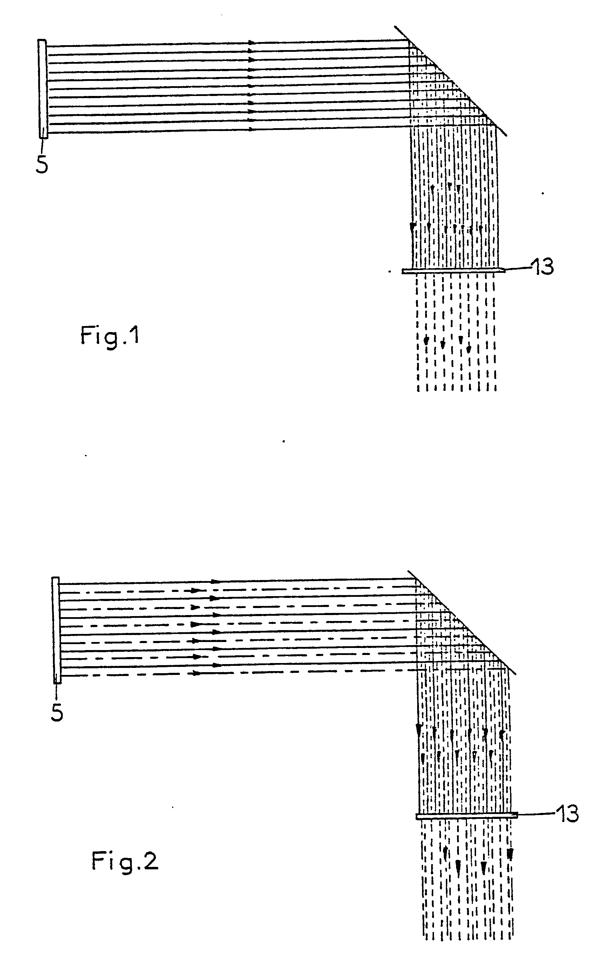

En fig. 1 est illustrée l'invention.

Pour former une image de l'oeil à photographier et/ou à observer sans apparition de reflets dus à une source d'éclairage en lumière ultraviolette laquelle lumière excite une substance fluorescente instillée dans l'oeil on ne sélectionne que les rayons lumineux propres à la substance fluorescente, c'est-à-dire les rayons lumineux émis par cette dernière lorsqu'elle est excitée par la substance fluorescente.

A cet effet, les longueurs d'ondes des rayons représentés en traits pleins sur la fig. 1 émis par la source d'éclairage en lumière ultraviolette sont inférieures ou égales à une valeur λ 1 propre à l'excitation de la substance fluorescente employée et correspondant à la plus petite longueur d'onde du rayonnement émis par celle-ci.

Les rayons émis par la substance fluorescente sont représentés en traits pointillés sur la fig.1.

Cette caractéristique a pour but d'éviter l'interférence des deux rayonnements ou des deux gammes de rayons émis respectivement par la source d'éclairage en lumière ultraviolette et par la substance fluorescente pour éliminer comme on le verra plus avant la totalité des reflets.

Dans la pratique pour un examen on emploie de préférence une substance fluorescente connue sous le nom de fluoreceine dont la longueur d'onde λ1 du rayonnement qu'elle émet est sensiblement égale ou supérieure à 500 nm.

La plage des rayons issus de l'oeil est constituée par les rayons émis par la substance fluorescente et par la gamme des rayons lumineux réfléchis et/ou diffusés sur l'oeil et/ou sur la lentille de contact.

Pour éliminer les reflets de la source d'éclairage en lumière ultraviolette susceptibles de masquer sur l'image de l'oeil des zones nécessaires à une analyse correcte du résultat de l'examen, les rayons issus de l'oeil sont filtrés pour ne sélectionner que ceux dont la longueur d'onde est supérieure à λ 1 et donc pour ne sélectionner que ceux propre à la substance fluorescente.

Comme on le comprend les rayons lumineux de la source d'éclairage en lumière ultraviolette réfléchis sur l'oeil et sur la lentille de contact se caractérisant chacun par une longueur d'onde inférieure à 1 sont donc supprimés par le filtrage.

En outre cette disposition a également pour but une amélioration du contraste entre les zones qui en sont exemptes donc sombres en permettant l'extinction complète de la lumière de la source d'éclairage réfractée par l'oeil et/ou par la lentille de contact.

Par analyse d'une image de l'oeil, il est parfois nécessaire d'éclairer les zones dépourvues de substance fluorescente sans pour autant réduire le contraste et de préférence sans introduire des reflets sur l'image. A cet effet l'invention illustrée schématiquement en fig.2 consiste à injecter dans les rayonnements émis par la source d'éclairage un faisceau de lumière blanche (représenté en traits mixtes fins).

La lumière blanche en très faible quantité augmente la luminosité de la substance fluorescente par apport de rayons lumineux de longueurs d'ondes supérieure à 1 et éclaire les zones de l'oeil qui en sont dépourvues comme les paupières et les régions sclérales, sans pour autant entraîner une diminution du contraste entre ces deux zones.

Comme on le comprend aisément, l'apport de lumière blanche permet la mise en évidence des nuances colorées de l'oeil.

C'est ainsi par exemple que les vaisseaux scléraux apparaîtront sensiblement suivant leur couleur naturelle sur l'image de l'oeil à observer et/ou à photographier.

En outre, l'apport de lumière blanche permet la mise en evidence du détail des zones pourvues de substance fluorescente.

Pour supprimer les reflets du faisceau de lumière blanche sur l'oeil et sur la prothèse et visibles sur l'image de l'oeil à photographier et/ou à observer, celui-ci est polarisé avant et après sa réflexion suivant deux plans correctement orientés.

L'expérience a montré que ces deux plans doivent être perpendiculaires l'un à l'autre pour l'exemple d'application décrit dans le présent brevet.

La seconde polarisation du faisceau peut être effectuée avant ou après filtrage des rayons lumineux issus de l'oeil.

Il est à noter que l'extinction des reflets du faisceau de lumière blanche au niveau de l'image de l'oeil ne nuit aucunement à sa diffusion.

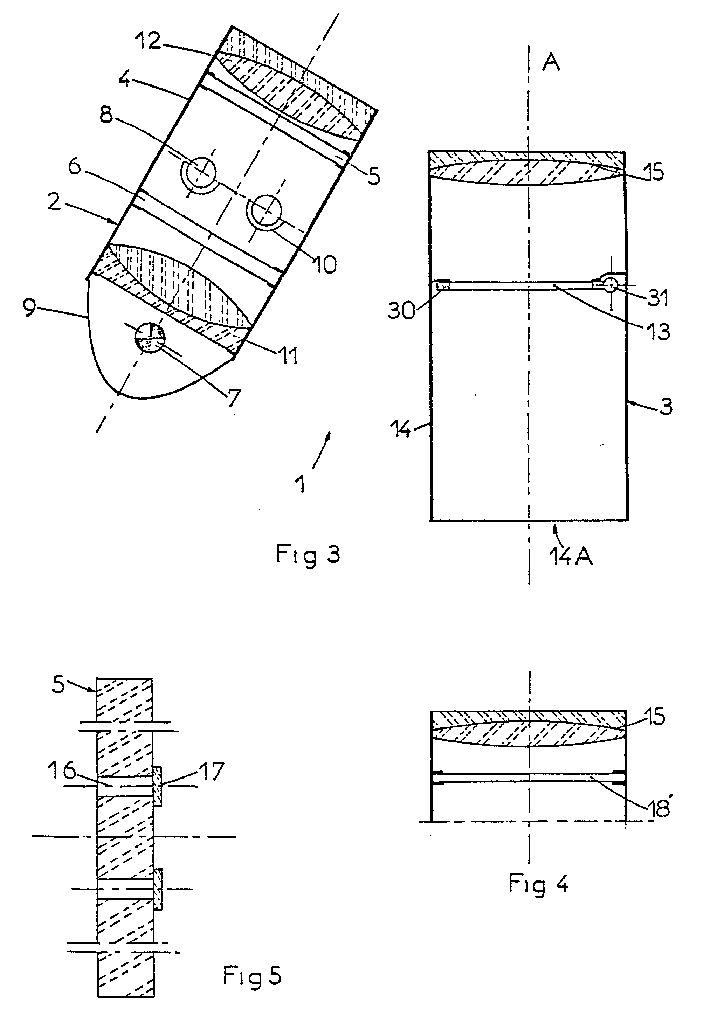

Tel que représenté schématiquement en fig.3, le dispositif 1 selon l'invention est constituée par au moins une source d'éclairage en lumière ultraviolette 2 d'émission de rayons ultraviolets et visibles de longueur d'onde inférieure à λ 1 et par un dispositif d'observation pour la formation de l'image de l'oeil à photographier et/ou à observer associé à un appareil de prise de vue et/ou un dispositif d'observation.

La source d'éclairage 2 en lumière ultraviolette dirigée vers l'oeil à photographier et/ou à observer comprend une monture 4 dans laquelle sont montés au moins un moyen d'émission de lumière blanche, au moins un organe 5 de sélection des rayons lumineux de longueur d'onde inférieure à λ 1, au moins un système de focalisation et au moins un organe d'arrêt 6 aux rayons calorifiques émis par le moyen 7.

Comme on peut le voir à l'examen de la fig.3, la source d'éclairage 2 en lumière ultraviolette comporte deux moyens 7 et 8 d'émission de lumière blanche constituée respectivement par une lampe à halogène et par un tube à éclat alimenté en énergie électrique lors de la prise de vue de manière connue par le mécanisme du boîtier de l'appareil photographique.

La lampe à halogène alimentée en courant basse tension par un réseau de conducteurs est affectée principalement à l'éclairage de l'oeil lors de son observation, mais de préférence demeure sous tension lors de la prise de vue photographique pour des raisons de simplification de montage.

Il est à noter que pour les prises de vue cadencée seule cette lampe 7 sera mise sous tension.

La lampe 7 et le tube à éclat 8 sont associés de manière connue chacun à un miroir dirigeant la majeure partie des rayons émis vers l'organe de sélection 5 des rayons de longueurs d'ondes inférieures à λ 1.

La lampe 7 et son miroir parabolique 9 sont montés en extrémité de la monture 4 de préférence en dehors de celle-ci pour faciliter l'échange thermique avec le milieu extérieur.

De préférence le tube à éclat 8 du type de ceux se présentant sous la forme d'un élément tubulaire cylindrique conformé en U est disposé avec son miroir 10 à l'intérieur de la monture 4 de telle sorte que le plan du U soit perpendiculaire à l'axe longitudinal de la source d'éclairage en lumière ultraviolette 2 et que les branches du dit U soient disposées de part et d'autre de cet axe pour des raisons que seront exposées plus avant.

Les rayons lumineux issus de la lampe 7 et/ou du tube à éclat 8 sont filtrés par l'organe 5 qui constitue un écran aux rayons de longueurs d'ondes supérieures à λ 1.

L'organe 5 ou filtre disposé sur le trajet des rayons lumineux est de préférence monté en extrémité de la monture 4 en regard de l'oeil à examiner. Selon un exemple préférentiel de réalisation, l'organe 5 est un filtre ne laissant pàsser que les rayons visibles et invisibles de longueur d'ondes inférieures à 500 nm.

Pour éviter la présence d'ombres sur la zone à photographier par exemple dues à l'interception des rayons lumineux issus de la lampe 7 par le tube à éclat 8 et pour concentrer la totalité des rayons de longueurs d'ondes inférieures à λ 1 sur la dite zone la source d'éclairage en lumière ultraviolette 2 est dotée d'un système optique de focalisation.

Selon l'exemple préférentiel de réalisation représenté schématiquement en fig.3, le système optique de focalisation est constitué par un premier jeu 11 de lentilles situé sur le trajet des rayons lumineux de la lampe 7 et par un second jeu 12 de lentilles disposé sur le trajet des rayons lumineux issus de la source d'éclairage en lumièr ultraviolette. Le jeu 11 de lentilles associé au miroir 9 de la lampe 7 a pour but de focaliser les rayons lumineux issus de la lampe 7 et/ou réfléchis sur le dit miroir 9 en un point situé sur l'axe de la source d'éclairage en lumière ultraviolette 2, de préférence entre les branches du tube à éclat 8.

Il est évident que la position du point de focalisation sur l'axe n'est donné ici qu'à titre indicatif, le but à atteindre étant comme on le comprend d'écarter du tube à éclat l'enveloppe conique des rayons issus de la lampe 7.

Le jeu 12 de lentilles disposé sur le trajet des rayons lumineux issus de la source d'éclairage en lumière ultraviolette 2 évite la dispersion de ceux-ci en les concentrant sur la zone de l'oeil à photographier et/ou à observer.

De préférence, le jeu de lentille 12 focalise les rayons issus de la source d'éclairage en lumière ultraviolette 2 en arrière de la zone observée, mais peut selon une variante de réalisation les focaliser en avant.

Selon un autre exemple de réalisation, le jeu de lentilles 12 peut être remplacé par un système optique permettant la variation du point de focalisation des rayons issus de l'oeil pour éclairer des zones d'observation de différentes étendues.

Comme connu la source d'éclairage en lumière ultraviolette 2 est dotée d'un organe d'arrêt des rayons calorifiques émis par la lampe 7 constitué par un filtre catathermique 6 disposé dans la monture entre le jeu de lentille 11 et le tube à éclat 8 ou entre la lampe 7 et le dit jeu.

Pour renforcer cette caractéristique, le miroir 9 est du type dicroique permettant la réfraction des rayons calorifiques.

Comme dit précédemment les rayons de longueurs d'ondes inférieure à λ 1 excitent une substance fluorescente instillée dans les larmes de l'oeil qui émet une gamme de rayons lumineux de longueur d'onde supérieure à λ 1.

Pour sélectionner les rayons propres à la substance fluorescente et pour supprimer les reflets de la source d'éclairage en lumière ultraviolette 2 sur l'oeil et/ou sur la prothèse, le dispositif d'observation 3 du dispositif 1 est équipé d'un filtre 13 sélectionnant les rayons de longueurs d'ondes supérieur à λ 1.

Le dispositif d'observation 3 dirigé vers l'oeil à photographier et/ou à observer est constitué par une monture 14 dans laquelle sont disposés un ou plusieurs jeux de lentilles 15 pour la formation de l'image de l,oeil.

A la monture 14 sont associés un boîtier d'un appareil photographique et/ou un dispositif d'observation connu en soi de préférence monté sur glissières pour venir se disposer séparément en regard de l'orifice 14A de la monture.

En arrière du ou des jeux de lentilles 15 et sur le trajet des rayons lumineux issus de l'oeil est disposé dans la

4

monture le filtre 13 lié à celle-ci par tous moyens connus.

Selon une autre forme de réalisation le dispotif selon l'invention est équipé d'au moins un moyen d'emission d'un faisceau de lumière blanche conjointement à l'émission des rayons de longueur d'ondes inférieures à λ1 pour éclairer les zones de l'oeil exemptes de substances fluorescentes et pour augmenter la luminosité de ladite substance. Avantageusement, le moyen d'émission du faisceau de lumière blanche est constitué par la lampe 7 et/ou par le tube à éclat 8.

A cet effet, comme représenté en fig.4, le filtre 5 de sélection des rayons de longueur d'onde inférieure à λ1 est doté d'au moins un orifice transversal 16 de petite dimension pour ne laisser passer qu'une très faible quantité de lumière blanche.

Pour supprimer la présence des reflets visibles sur l'image de la zone à photographier et/ou à observer dus à la reflexion du faisceau de lumière blanche sur l'oeil et/ou sur la lentille de contact celui-ci est polarisé avant et après sa reflexion par deux filtres polarisant 17 et 18 disposés respectivement dans la source d'éclairage 2 et dans le dispositif d'observation 3.

Comme on peut le voir en fig. 4 et 5, le filtre 17 de dimension légèrement supérieure au diamètre de l'orifice 16 est fixé sur une des faces du filtre 5 en regard du dit orifice.

Le filtre 18 du dispositif d'observation 3 est de préférence disposé dans la monture à la suite du jeu de lentille 15 et son plan de polarisation est perpendiculaire à celui du filtre 17 pour supprimer sur 1,image de l'oeil les reflets dus au faisceau de lumière blanche.

Avantageusement la zone de l'oeil à examiner est éclairée suivant plusieurs angles pour assurer une répartition égale de la lumière.

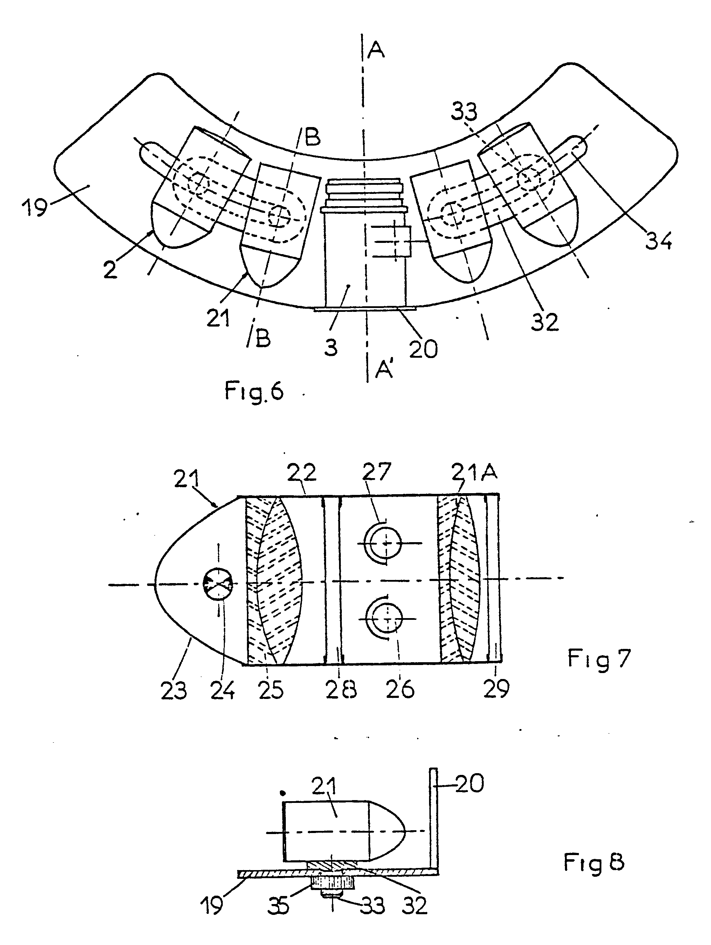

A cet effet, selon une forme préférentielle de réalisation, le dispositif 1 est équipé de deux sources d'éclairage en lumière ultraviolette alimentées par un même circuit électrique disposées de part et d'autre de l'axe AA' du dispositif d'observation 3 et à égale distance de l'oeil à examiner comme représenté en Fig. 6.

A l'examen de cette figure on peut voir que les sources d'éclairage en lumière ultraviolette 2 et le dispositif d'observation 3 sont montés sur une platine horizontale 19 épousant pour des raisons d'encombrement la forme d'un secteur de couronne.

La platine 19 est pourvue d'une aile verticale 20 dirigée vers le haut à laquelle se fixent la monture 14 du dispositif d'observation 3 par tous moyens connus et les glissières non représentées de l'appareil photographique et/ou d'observation.

Pour permettre la prise photographique et/ou l'observation de l'oeil en Lumière blanche, le dispositif 1 est équipé d'au moins une source 21 d'éclairage de lumière blanche et de moyens permettant le retrait du filtre 13 du dispositif 3 du trajet des rayons lumineux issus de l,oeil.

Avantageusement, pour des raisons précédemment énoncées, le dispositif 1 est équipé de deux sources 21 de lumières blanches alimentées par un même circuit électrique disposées de part et d'autre de l'axe AA' du dispositif d'observation 3 entre celui-ci et les sources d'éclairage en Lumière ultraviolette 2.

Comme on peut le voir en fig.7, chaque source d'éclairage en lumière blanche 21 comporte une monture 22, un miroir parabolique 23 associé à une lampe à halogène 24, un jeu de lentille 25 pour focaliser les rayons émis par la lampe 24 entre les branches d'un tube à éclat 26 associé à un miroir 27, un filtre catathermique 28 et un diffuseur 29 connu en soi permettant une répartition uniforme de la lumière sur l,oeil à examiner.

L'action des deux diffuseurs 29 et l'éclairage sous deux angles différents permet la suppression sur l'oeil des ombres.dues par exemple aux paupières, aux cils, etc...

En outre, le diffuseur n'altère pas les caractéristiques de la lumière blanche émise, ce qui permet la distinction des nuancés colorées de l'oeil et la détection des anomalies.

Avantageusement, chaque source d'éclairage en lumière blanche 21 est équipée d'un jeu de lentilles 21A avant ou après le diffuseur pour concentrer les rayons issus de la dite source.

Lors d'un examen en lumière blanche le filtre 13 ne doit pas intercepter les rayons lumineux issus de l'oeil.

A cet effet, comme représenté schématiquement en fig.3, le filtre 13 est monté sur un volet 30 mobile dans la monture 14 autour d'un axe perpendiculaire à l'axe AA' pour venir se disposer parallèlement au trajet des rayons lumineux et contre la paroi interne de la dite monture. Le volet 30 est par exemple constitué par un cadre sur lequel se fixe la bordure du filtre, comportant dans le prolongement d'un de ses bords une tige cylindrique 31 montée articulée dans des orifices de la monture 14.

Une des extrémités de la tige 31 coopère en dehors de la monture et sous la platine 19 avec un bouton molleté non représenté pour la commande du volet mobile.

En fonctionnement les sources d'éclairage en lumière blanche 21 occasionnent des reflets sur l'oeil et/ou sur la lentille de contact visible sur l'image de l'oeil.

Ceci n'est pas génant dans la mesure où les reflets se situent en dehors de la zone à examiner de l'oeil.

En fonction de la zone choisie pour l'examen, les sources d'éclairage en lumière blanche 21 sont susceptibles d'etre écartées ou rapprochée l'une de l'autre tout en restant à égale distance de l'oeil.

Pour éviter que la position dés reflets ne varie lors de la prise photographique, la lampe 24 est coaxiale à l'axe de symétrie de la source d'éclairage en lumière blanche 21 et les branches du tube à éclat sont disposées symétriquement de part et d'autre de cet axe.

Avantageusement cette caractéristique est reproduite sur la source d'éclairage en lumière ultraviolette 2 pour permettre la localisation des reflets du faisceau de lumière blanche lorsque celui-ci n'est pas polarisé.

Selon l'exemple préférentiel de réalisation représenté en fig. 6, les sources d'éclairage 21 en lumière blanche sont montées chacune sur un support 32 mobile suivant un arc de cercle par rapport à la platine 19. Ce support 32 est par exemple constitué par une plaque métallique pourvue de deux pions de guidage 33 engagés dans une même rainure 34 en arc de cercle de la platine 19 centrée par rapport à l'oeil en sorte que au cours du mouvement angulaire d'écartement ou de rapprochement des sources celles-ci soient toujours dirigées vers un même point de l'axe AA'.

Un des pions de guidage de chaque support est fileté pour recevoir un bouton molleté 35 de blocage en position de la source d'éclairage en lumière blanche 21 par rapport à la platine 13.

Avantageusement, les sources 2 d'éclairage en lumière ultraviolette sont fixées chacune à un support 32 pour accroître l'écartement des sources d'éclairage en lumière blanche 21 et pour localiser les reflets dans le cas précédemment énoncé.

Les reflets sur l'oeil et/ou sur la prothèse oculaire des sources d'éclairage en lumière blanche 21 peuvent être atténués comme connu par des filtres polarisants disposés respectivement sur le trajet des rayons rayons lumineux issus de ces sources d'éclairage en lumière blanche 21 et sur le trajet des rayons lumineux pénétrant dans le dispositif d'observation 3.

Avantageusement le filtre 18 de la forme de réalisation des fig. 4 et 5 peut trouver son emploi également dans la polarisation des rayons lumineux issus de l'oeil lorsque celui-ci est éclairé en lumière blanche.

Selon une variante de réalisation, les filtres polarisants sont montés chacun sur un volet mobile devant les sources d'éclairage en lumière blanche 21 et le dispositif 3, pour intercepter ou non le trajet des rayons lumineux.

Les sources d'éclairage en lumière ultraviolette 2 et les sources d'éclairage en lumière blanche 21 sont montées en série respectivement sur deux circuits indépendants mis sous tension successivement par un commutateur à deux positions.

Avantageusement ce commutateur est constitué par deux boutons poussoirs commandés l'un après l'autre par une came montée sur la tige 31 en dehors de la monture 14.

Lorsque le filtre 13 du dispositif d'observation 3 est perpendiculaire à l'axe AA' la came agit seulement sur le bouton poussoir de mise sous tension du circuit électrique de commande des sources d'éclairage en lumière ultraviolette 2.

Inversement lorsque le filtre 13 est parallèle à l'axe AA' la came agit seulement sur le bouton poussoir de mise sous tension du circuit électrique de commande des sources d'éclairage en lumière blanche 21.

Chaque circuit de commande comprend une alimentation basse tension des lampes 7 ou 24 et un système électrique synchronisé à l'obturateur du boîtier photographique pour le déclenchement des tubes à éclat 8 ou 26.

Le dispositif 1 tel que décrit est protégé par une coque par exemple en polyester fixée à la platine 19 par tous moyens connus. Au moins une des parois de la coque est percée d'orifices d'évacuation de la chaleur dégagée par les sources d'éclairage 2 et 21.

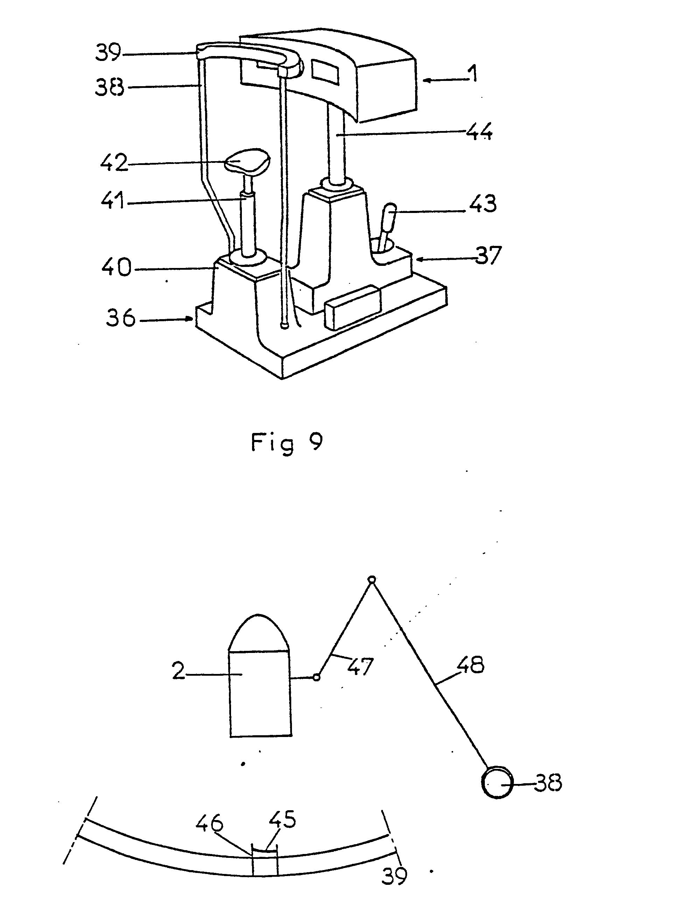

Comme représenté en fig.9, le dispotif 1 précédemment décrit est associé à des moyens de positionnement du dispositif d'observation 3 et des sources d'éclairage en lumière ultraviolette 2 et en lumière blanche 21 en vis à vis de l'oeil à examiner.

Ces moyens sont constitués par un bâti 36 reposant sur un plan de travail et par un chariot 37 mobile portant le dispositif 1. Le bôti 36 par exemple rectangulaire est pourvu d'une armature dotée de quatre organes élastiques d'appui au plan de travail, recouverte d'un habillage constitué de préférence par une coque en polyester.

A ce bôti sont fixés deux montants 38 sensiblement verticaux portant en extrémité haute un appui tête 39 cintré disposé dàns un plan horizontal.

Entre les montants 38 est fixé à un boîtier 40 du bôti un arbre vertical télescopique portant en extrémité une mentionnière 42. Dans le boîtier 7 sont montés différents organes électriques d'alimentation des sources d'éclairage en lumière ultraviolette 2 ou des sources d'éclairage en lumière blanche 21.

Au moins une des parois de ce boîtier est percée d'orifices d'évacuation de la chaleur dégagée par les organes électriques.

En arrière du boîtier 40, le bôti est doté de moyens connus assurant le guidage du chariot mobile 37 suivant deux directions perpendiculaires d'un plan horizontal pour disposer le dispositif 1 en regard de l'oeil à examiner et pour régler sa distance à cet oeil.

Avec les moyens de guidage coopère une poignée 43 du chariot montée comme connue sur une rotule.

Le chariot mobile 37 est pourvu d'une colonne verticale de préférence téléscopique 44 solidaire de la platine 19 du dispositif 1.

1. Dispositif pour observer et/ou photographier l'oeil d'un patient en vue de l'adaptation d'une lentille de contact comportant un dispositif d'observotion (3) pour observer et/ou photographier l'oeil, une source (21) de lumière blanche pour éclairer l'oeil en lumière blanche et une source (2) de lumière ultraviolette pour éclairer l'oeil en lumière ultraviolette lorsque dans celui-ci est instillé une substance fluorescente qui, lorsqu'elle est exitée par la lumière ultraviolette, émet des rayons lumineux dont la longueur d'ande est supérieure à une valeur $\lambda 1$ égale à 500 nm, ladite source (2)de lumière ultraviolette étant constituée notamment par une lampe (7), par un tube à éclat (8) et par un filtre ultraviolet (5) qui ne laisse passer que la lumière ultraviolette, caractérisé en ce que le dispositif d'observation (3) est équipé d'un filtre (13) qui ne selectionne que les rayons issus de l'oeil dont la longueur d'onde est supérieure à la valeur $\lambda 1$.

2. Dispositif selon la revendication 1 pour observer et/ou photographier l'oeil d'un patient, comportant un dispositif d'observation (3) et une source de lumière ultraviolette dotée notamment d'un filtre ultraviolet (5) caractérisé en ce que le filtre (5) est percé d'au moins un orifice (16) pour permettre le passage d'un faisceau de lumière blanche non filtrée, ce dit faisceau étant polarisé avant sa reflexion sur l'oeil par un filtre polarisant (17) disposé sur une des faces du filtre (5) en regard de l'orifice (16) et étant polarisé après sa reflexion sur l'oeil par un filtre polarisant (18) disposé dans le dispositif d'observation (3).

3. Dispositif selon la revendication 1 comportant une source (2) de lumière ultraviolette qui est munie d'une lampe (7) et d'un tube à éclat (8) en forme de U, caractérisé en ce que le dit tube à éclat (8) est disposé en sorte que le plan dans lequels se trouvent les axes de ses branches, soit disposé perpendiculairement à l'axe longitudinal de la dite source et en sorte que les branches du U que forme ce tube soient disposées de part et d'autre de cet axe, et en ce que la dite source de lumière ultraviolette comporte un jeu de lentilles (11) pour focaliser la lumière émise par la lampe (7) entre les branches du U, ce qui permet d'éviter toutes ombres sur l'oeil, ces ombres étant dues à la présence du tube à éc1at,et de selectionner les rayons émis par la substance fluorescente.

4. Dispositif de formation d'une image de l'oeil selon la revendication 1, caractérisé en ce que le filtre (13) est monté sur un volet mobile (30) muni d'un axe d'articulation (31) à la monture pour se disposer suivant une position perpendiculaire ou parallèle au trajet des rayons lumineux dans le dispositif d'observation (3).

5. Dispositif de formation d'une image de l'oeil exempte de reflets selon la revendication 1 caractérisé en ce qu'il est muni de deux sources (2) de lumière ultraviolette groupées chacune à une source de lumière blanche (21) sur un support (32) et situées de part et d'autre de l'axe AA' du dispositif d'observation (3).

6. Dispositif pour la formation d'une image de l'oeil selon la revendication 4, caractérisé en ce que chaque support (32) est susceptible de coulisser dans une lumière (34) en arc de cercle, centrée par rapport à l'oeil à examiner et ménagée dans une platine (19) du dispositif, pour écarter ou rapprocher la source de lumière blanche (21) et la source (2) de lumière ultraviolette de l'axe AA'.

7. Dispositif pour la formation d'une image de l'oeil selon la revendication 4 comportant deux sources (21) de lumière blanche, caractérisé en ce que chacune des deux sources est munie d'un diffuseur (29).


## ANSPRÜCHE

1. Gerät zur Beobachtung und/oder zum Photographieren des Auges Eines Patienten vor der Anpassung einer Kontaktlinse, das aus einer Beobachtungsvorrichtung (3) zur Beobachtung und/oder zum Photographieren des Auges, einer Weisslichtquelle (2A) zur Beleuchtung des Auges mit Weisslicht und einer Ultraviolettlichtquelle (2) zur Beleuchtung des Auges mit Ultraviolettlicht besteht, wenn das Auge einen Lenchstoff durch Eintropfeln bekommen hat, welcher unter Anregung durch das Ultraviolettlicht Lichtstrahlen sendet, deren Wellenlange hoher als ein Wert $\lambda$ 1 von 500 nm ist, die vorgenannte Ultraviolettlichtquelle (2) besteht namentlich aus einer Lampe (7), einer Blinkzeichenröhre (8) und einem Ultraviolettfilter (5), der nur das Ultraviolettlicht durchlässt, dadurch gekennzeichnet dass, das Beobachtungsgerät (3) mit einem Filter (13) versehen ist, der nur das vom Auge gestrahlte Licht durchlässt, dessen Wellenlänge höher als der Wert $\lambda$ 1 ist.

2. Gerät nach Anspruch 1, zur Beobachtung und/oder Photographieren des Auges eines Patienten, das aus einer Beobachtungsvorrichtung (3) und einer mit einem Ultraviolettfilter (5) versehener Ultraviolettlichtquelle beteht, dadurch gekennzeichnet, dass der Filter mit mindestens einem Loch zur Durchlassung eines nicht gefilterten Weisslichtstrahl versehen ist, dieser Strahl wird vor der Rückstrahlung aufs Auge durch einen sich auf einer Seite des Filters (5) gegenüber dem Lich (16) befindenden Polarisationsfilter (17) polarisiert und nach der Rückstrahlung aufs Auge durch einen sich in der Beobachtungsvorrichtung (3) befindenden Polarisations-filter (18) polarisiert.

3. Gerät nach Anspruch 1, das aus einer mit einer Lampe (7) und einer U-formigen Blinkzeichenröhre (8) versehenen Ultraviolettlichtquelle besteht, dadurch gekennzeichnet, dass die sogenannte Blinkzeichenröhre (8) so angesetzt ist, dass die Fläche, auf der die Achsen inrer Schenkel sich befinden, senkrecht zu der Längschse der genannten Quelle angesetzt ist, und dass die u-Schenkel der Blinkzeichenröhre auf beiden Seiten dieser Axe sich befinden, dass die voragenannte Ultraviolettlichtquelle einer Linsensat (11) umfasst, der das von der Lampe (7) gesandte Licht zwischen den USchenkeln führt und dadurch ermöglicht, die Erscheinung aufs Auge der durch die Blinkzeichenröhre eingeführten Schatten zu vermeiden und die durch den Leuchstoff gesandten Strahlen auszuwählen.

4. Gerät zur Augenbildabbildung nach Anspruch 1, dadurch gekennzeichnet, dass der Filter (13) auf einer mit einer Drehachse (31) am Beschlag versehenen beweglichen Klappe eingebaut ist und ist sekrecht oder parallel zum Verlauf der Lichtstrahlen in der Beobachtungsvorrichtung angesetzt.

5. Gerät zur reflexfreien Augenbildabbildung nach Anspruch 1, dadurch gekennzeichnet, dass das Gerät aus zwei Ultraviolettlichtquellen (2) besteht, die jeweils mit einer Weisslichtquelle (21) auf einem Träger (32) verbunden sind und sich beiderseits der Achse AA' der Beobachtungsvorrichtung (3) befinden.

6. Gerät zur Augenbildabbildung nach Anspruch 4, dadurch gekenzeichnet, dass jeder Träger in einem kreisförmigen Lichtbogen (34) aufschiebbar ist, der gegenüber dem zu beobachtenden Auge zentral ausgerichtet ist und auf einer Halteplatte (19) der Vorrichtung angeordnet ist, um die Weisslichtquelle (21) und die Ultraviolettlichtquelle (2) der Achse AA' zu entfernen oder nähern.

7. Gerät zur Augenbildabbildung nach Anspruch 4, der aus zwei Weisslichtquellen besteht, dadurch gekennzeichnet, dass jede Lichtquelle mit einem Diffusor (29) versehen ist.

## Claims

1. Device for observing and/or photographing the eye of a patient in order to fit a contact lens comprising an observation device (3) for observing and/or photographing the eye, a white light source (21) for lighting the eye with white light and an ultraviolet light source (2) for lighting the eye with ultraviolet light when eye has received a fluorescent substance by instillment which, when excited by the ultraviolet light, emits light rays with wavelength greater than a value $\lambda$ equal to 500 nm, the said source (2) of ultraviolet light being comprised mainly by a lamp (7), a flash tube (8) and an ultraviolet filter (5) which only lets through the ultraviolet light, characterized in that the observation device (3) is equipped with a filter (13) which only selects the rays delivered by the eye the wavelength of which is greater than value $\lambda$ 1.

2. Device according to claim 1 for observing and/or photographing the eye of a patient, comprising an observation device (3) and an ultraviolet light source mainly equipped with an ultraviolet filter (5) characterized in that filter (5) is drilled with at least one hole (16) enabling the passage of a non filtered white light beam, this said beam being polarized before its reflection on the eye by a polarizing filter (17) placed on one of the faces of the filter (5) opposite the hole (16) and being polarized after its reflection on the eye by a polarizing filter (18) placed in the observation device (3).

3. Device according to claim 1 comprising an ultraviolet light source (2) equipped with a lamp (7) and a U shaped flash tube (8), characterized in that the said flash tube (8) is positioned so that the plane, in which lie the axes of its branches, is positioned perpendicularly to the horizontal axis of the said source and so that the branches of the U forming this tube are placed on either side of this axis, and in that the said ultraviolet light source comprises a set of lenses (11) for focusing the light emitted by lamp (7) between the U branches, thus avoiding all shadows on the eye, these shadows being due to the presence of the flash tube, and to select the emitted rays by the fluorescent substance.

4. Eye image forming device according to claim 1, characterized in that filter (13) is installed on a movable flap (30) equipped with a hinge pin (31) on mounting for arrangement following a position perpendicular or parallel to the path of the light rays in the observation device (3).

5. Eye image forming device free from réflections according to claim 1 characterized in that it is equipped with two ultraviolet light sources (2) each one associated with a white light source (21) on a support (32) and located on either side of axis AA' of the observation device (3).

6. Eye image forming device according to claim 4, characterized in that each support (32) is capable of sliding through a light (34) in the form of the arc of a circle, centered with respect to the eye under examination and arranged in a support plate (19) of the device, for moving the white light source (21) nearer to or further away from the ultraviolet light source (2) of axis AA'.

7. Eye image forming device according to claim 4 comprising two white light sources (21), characterized in that each of the two sources is equipped with a diffuser (29).

Fig.1

Fig.2

Fig 3

Fig 4

Fig 5

Fig.6

Fig 7

Fig 8

Fig 9